# EUROPEAN PATENT APPLICATION

(11) **EP 3 709 015 A2**
(43) Date of publication of application: **16.09.2020**
(21) Application number: 20160633.2
(22) Date of filing: 03.03.2020
(51) Int. Cl.: G01N 33/24, G01H 1/00

(54) **SOIL CONDITION ESTIMATION APPARATUS, SOIL CONDITION ESTIMATION METHOD, AND PROGRAM**

(30) Priority: 14.03.2019 JP 2019046894
(71) Applicant: NEC Corporation, Tokyo 108-8001 (JP)
(72) Inventor: AZUMA, Tan, Minato-ku, Tokyo 1088001 (JP); KIKUCHI, Yusuke, Minato-ku, Tokyo 1088001 (JP); ISHIDA, Kousuke, Minato-ku, Tokyo 1088001 (JP); AKIMOTO, Shunsuke, Minato-ku, Tokyo 1088001 (JP); FUJIYAMA, Kenichiro, Minato-ku, Tokyo 1088001 (JP); OOMINATO, Shinji, Minato-ku, Tokyo 1088001 (JP)
(74) Representative: Betten & Resch

(57) **Abstract**

A soil condition estimation apparatus 1 includes a selection unit 2 that, by using a propagation time that vibrations take to propagate through a soil, selects vibrations measured from the soil, according the depth of the soil; an extraction unit 3 that extracts a feature of the selected vibrations; and an estimation unit 4 that estimates conditions of the soil by referencing soil estimation information in which the feature and conditions of the soil are associated with each other, using the feature.

## Description

### INCORPORATION BY REFERENCE

This application is based upon and claims the benefit of priority from Japanese patent application No.2019-046894, filed on March 14, 2019, the disclosure of which is incorporated herein in its entirety by reference.

### BACKGROUD OF THE INVENTION

### 1. Technical Field

The present invention relates to a soil condition estimation apparatus, a soil condition estimation method, and a program for estimating soil conditions.

### 2. Background Art

A method for measuring the hardness of soil by using a hardness tester is known. For example, Japanese Patent Laid-Open Publication No. H11-94732 discloses a soil surface hardness measurement apparatus for measuring the hardness of a soil surface (ground surface). This soil surface hardness measurement apparatus drops a cone-shaped sensor of which the penetration depth thereof changes depending on the hardness of a ground surface, so that the sensor falls freely and penetrates soil, and the hardness of the soil surface is measured based on the penetration depth of the sensor.

Also, WO 2016/136213 discloses a soil quality determination apparatus that determines the type and density of soil as a related technology. This soil quality determination apparatus measures the type and density of soil based on frequency characteristic vibrations, which indicate frequency characteristics of vibrations of soil, and the amount of water.

However, the soil surface hardness measurement apparatus disclosed in Japanese Patent Laid-Open Publication No. H11-94732 is an apparatus for measuring the hardness of a ground surface, and this apparatus cannot be employed when the hardness of soil inside the ground is to be measured. Also, this apparatus causes a cone-shaped sensor to freely fall onto a ground surface, and is therefore not suitable for measuring the hardness of a soft soil such as a soil of a farm field. This is because, if a ridge or the like has been formed in the field, the sensor may damage the ridge.

The soil quality determination apparatus disclosed in WO 2016/136213 can determine the type and density of soil without damaging the ground surface. However, the soil quality determination apparatus disclosed in WO 2016/136213 is not designed to estimate soil conditions at various depths.

### SUMMARY

An example object of the present invention is to provide a soil condition estimation apparatus, a soil condition estimation method, and a program for estimating soil conditions at various depths.

In order to achieve the aforementioned object, a soil condition estimation apparatus according to an example aspect of the present invention includes:
a selection unit that, by using a propagation time that vibrations take to propagate through a soil, selects vibrations measured from the soil, according the depth of the soil;
an extraction unit that extracts a feature of the selected vibrations; and
an estimation unit that estimates conditions of the soil by referencing soil estimation information in which the feature and conditions of the soil are associated with each other, using the feature.

Also, in order to achieve the aforementioned object, a soil condition estimation method according to an example aspect of the present invention includes:
(a) a step of, by using a propagation time that vibrations take to propagate through a soil, selecting vibrations measured from the soil, according the depth of the soil;
(b) a step of extracting a feature of the selected vibrations; and
(c) a step of estimating conditions of the soil by referencing soil estimation information in which the feature and conditions of the soil are associated with each other, using the feature.

Furthermore, in order to achieve the aforementioned object, a program including instructions that causes a computer to carry out:
(a) a step of, by using a propagation time that vibrations take to propagate through a soil, selecting vibrations measured from the soil, according the depth of the soil;
(b) a step of extracting a feature of the selected vibrations; and
(c) a step of estimating conditions of the soil by referencing soil estimation information in which the feature and conditions of the soil are associated with each other, using the feature.

As described above, according to the present invention, it is possible to estimate soil conditions according to the depth of soil.

### BRIEF DESCRIPTION OF THE DRAWINGS

FIG. 1 is a diagram showing an example of a soil condition estimation apparatus.
FIG. 2 is a diagram illustrating soil, and propagation time.
FIG. 3 is a diagram showing an example of a system that includes the soil condition estimation apparatus.
FIG. 4 is a diagram showing an example data structure of selection time period generation information.
FIG. 5 is a diagram illustrating a resonance frequency.
FIG. 6 is a diagram showing an example data structure of soil estimation information.
FIG. 7 is a diagram showing example operations of the soil condition estimation apparatus.
FIG. 8 is a diagram showing an example of a computer that realizes the soil condition estimation apparatus.

### EXEMPLARY EMBODIMENT

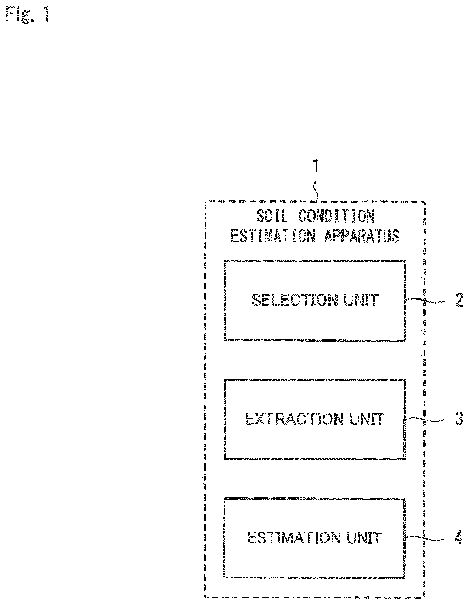

The following describes an exemplary embodiment of the present invention with reference to FIGS. 1 to 8.

### [Apparatus Configuration]

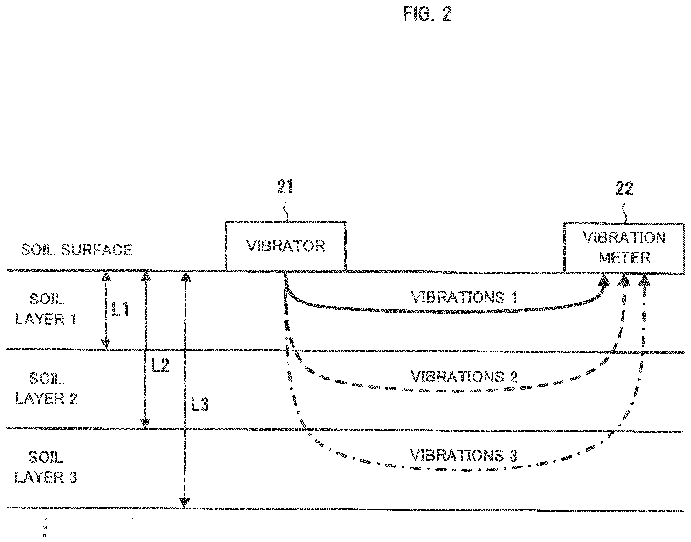

First, the configuration of a soil condition estimation apparatus 1 according to the present exemplary embodiment will be described with reference to FIGS. 1 and 2. FIG. 1 is a diagram illustrating an example of a soil condition estimation apparatus. FIG. 2 is a diagram illustrating soil, and propagation time.

The soil condition estimation apparatus 1 shown in FIG. 1 is an apparatus that estimates conditions of soil at various depths. As shown in FIG. 1, the soil condition estimation apparatus 1 includes a selection unit 2, an extraction unit 3, and an estimation unit 4.

The selection unit 2 selects, by using a propagation time, which is the time it takes for vibrations to propagate through soil, vibrations that have been measured from soil, according to the depth of soil. That is to say, the selection unit selects vibrations corresponding to the depth of soil, based on the fact that the propagation time varies depending on the depth of soil.

The depth of soil can be expressed as the distance thereof from the ground surface (soil surface) in a vertical direction, for example. In the example shown in FIG. 2, a soil that ranges from the soil surface to a distance L1 is referred to as a soil layer 1, a soil that ranges from the distance L1 to a distance L2 is referred to as a soil layer 2, and a soil that ranges from the distance L2 to a distance L3 is referred to as a soil layer 3.

The propagation time is, specifically, the period of time that elapses from when vibrations are applied to the ground surface of the subject soil to when vibrations corresponding to the applied vibrations are measured at the soil surface. As shown in FIG. 2, a vibrator 21 may be provided on the ground surface to apply vibrations to soil, for example. Also, as shown in FIG. 2, a vibration meter 22 may be provided on the soil surface to measure vibrations that have propagated to the soil surface as a result of the applied vibrations, for example.

The propagation time increases as the depth of soil increases. Therefore, as shown in FIG. 2, a relationship T3>T2>T1 is satisfied, where T1 denotes the propagation time of vibrations 1 (a solid line) that propagate to the vibration meter 22 through the soil layer 1, T2 denotes the propagation time of vibrations 2 (dotted line) that propagate to the vibration meter 22 through the soil layer 2, and the T3 denotes the propagation time of vibrations 3 (dashed-dotted line) that propagate to the vibration meter 22 through the soil layer 3.

The extraction unit 3 extracts a feature of the selected vibrations. A feature of vibrations is, for example, information that indicates a feature such as a resonance frequency.

The estimation unit 4 estimates soil conditions by referencing soil estimation information in which features and soil conditions are associated with each other, using the extracted feature. Soil conditions include, for example, the hardness of soil, the amount of roots contained in soil, the amount of water contained in soil, the composition of soil, and so on. However, soil conditions are not limited to the aforementioned conditions.

As described above, according to the present exemplary embodiment, it is possible to select vibrations according to the depth of soil, and therefore it is possible to estimate the conditions of soil at a desired depth (a soil layer), using the vibrations corresponding to the soil layer.

### [System Configuration]

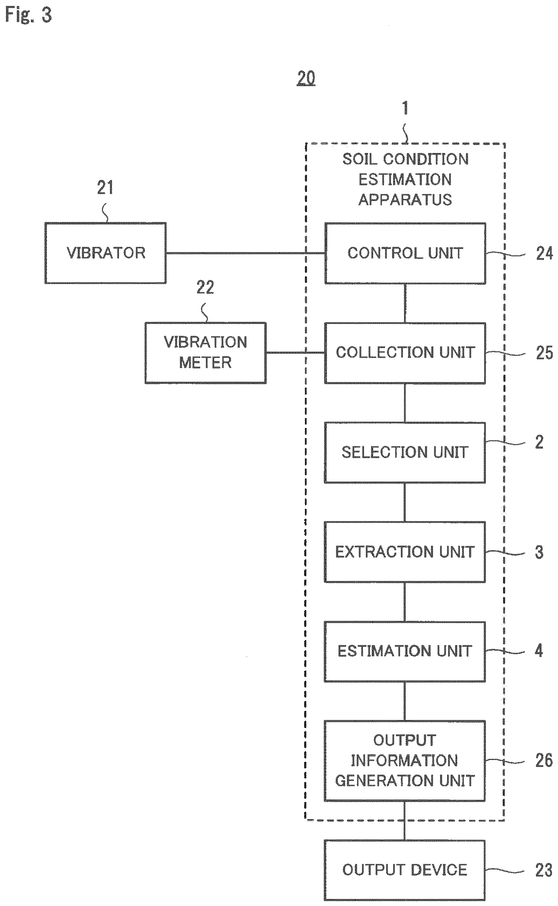

Next, the configuration of the apparatus 1 according to the present exemplary embodiment will be more specifically described with reference to FIG. 3. FIG. 3 is a diagram illustrating an example of a system that includes the soil condition estimation apparatus.

As shown in FIG. 3, a system 20 that includes the soil condition estimation apparatus 1 according to the present exemplary embodiment includes the vibrator 21, the vibration meter 22, and an output device 23 in addition to the soil condition estimation apparatus 1. The soil condition estimation apparatus 1 includes a control unit 24, a collecting unit 25, and an output information generation unit 26 in addition to the selection unit 2, the extraction unit 3, and the estimation unit 4.

The vibrator 21 is a device that is used to apply vibrations to soil. Specifically, the vibrator 21 is provided on the ground surface of soil, and applies vibrations to the soil. The vibrator 21 first acquires, from the control unit 24, vibration setting information that is used to set vibrations that are to be applied by the vibrator 21. Vibration setting information is, for example, information that is used to set a vibration force and a vibration frequency, for example.

Next, the vibrator 21, upon acquiring vibration setting information from the control unit 24, causes soil to vibrate, based on the vibration setting information. Examples of vibration methods include a method through which the vibration frequency is swept so that vibrations are generated at a plurality of frequencies in the soil.

Note that the vibrator 21 may be a mechanical, hydraulic, electrodynamic, piezoelectric, or electromagnetic vibrator, or a hummer, for example. The vibrator 21 need only apply vibrations so as not to damage a ridge or the like formed on a soft soil such as a soil of a farm field. The vibrator 21 communicates with the control unit 24 via wireless communication, wired communication, or the like.

The vibration meter 22 is a device for measuring vibrations applied to soil. Specifically, the vibration meter 22 is provided on the ground surface, and measures vibrations applied by the vibrator 21 to soil, from the soil. Next, the vibration meter 22 outputs vibration information that indicates the measured vibrations, to the collecting unit 25.

The vibration meter 22 may be a mechanical, electromagnetic, piezoelectric, optical, or electromagnetic vibration meter, for example. The vibration meter 22 communicates with the control unit 25 via wireless communication, wired communication, or the like.

The output device 23 acquires output information that has been converted by the output information generation unit 26 so as to be in an outputtable format, and outputs an image, a sound, or the like that is generated based on the output information. The output device 23 is, for example, an image display device that employs a liquid crystal, organic EL (Electro Luminescence), or a CRT (Cathode Ray Tube). The image display device may also include a sound output device such as a speaker. The output device 23 may be a printing device such as a printer. Output information will be described later.

The following describes the soil condition estimation apparatus.

The control unit 24 controls at least the vibrator 21 and the collecting unit 25. Specifically, the control unit 24 performs control to transmit vibration setting information to the vibrator 21 and cause the vibrator 21 to apply vibrations. Also, the control unit 24 transmits a vibration start instruction to the collecting unit 25 and causes the collecting unit 25 to collect pieces of vibration information.

The collecting unit 25 collects pieces of vibration information from the vibration meter 22. Specifically, the collecting unit 25 first collects pieces of vibration information from vibration meter 22 in time series. Next, the collecting unit 25 outputs the pieces of vibration information thus collected to the selection unit 2. The collecting unit 25 also stores pieces of vibration information in a storage unit (not shown). The storage unit may be provided inside the soil condition estimation apparatus 1, or provided outside the soil condition estimation apparatus 1.

The selection unit 2 selects a piece of vibration information corresponding to the subject soil layer from among the pieces of vibration information collected by the collecting unit 25, based on the propagation time of vibrations corresponding to the depth of the subject soil (soil layer). Specifically, the selection unit 2 first acquires pieces of vibration information from the collecting unit 25.

Next, the selection unit 2 calculates a selection time period based on the propagation time corresponding to the subject soil layer. For example, when the piece of vibration information corresponding to the soil layer 1 is selected, the selection unit 2 calculates the time (selection time period) from when vibrations are applied by the vibrator 21 to soil to when the vibrations 1 that have been reflected by the soil layer 1 can be measured by the vibration meter 22, using the propagation time T1 corresponding to the soil layer 1.

That is to say, when the propagation time T1 is expressed as a period of time from a point in time t0, at which the ground surface of soil is caused to vibrate, to a point in time te1, at which the vibrations that have propagated through the soil layer 1 are measured at the ground surface, a selection time period ts1 corresponding to the soil layer 1 is a period of time from a point in time te1-a1, which is earlier than the point in time te1, to a point in time te1+b1, at which vibrations reflected by the soil layer 1 are no longer detectable.

Note that the time a1 (start information) and the time b1 (end information) can be obtained through experiments or simulations, for example. Similarly, selection time periods ts2, ts3, and so on are respectively calculated for the soil layers 2, 3, and so on other than the soil layer 1.

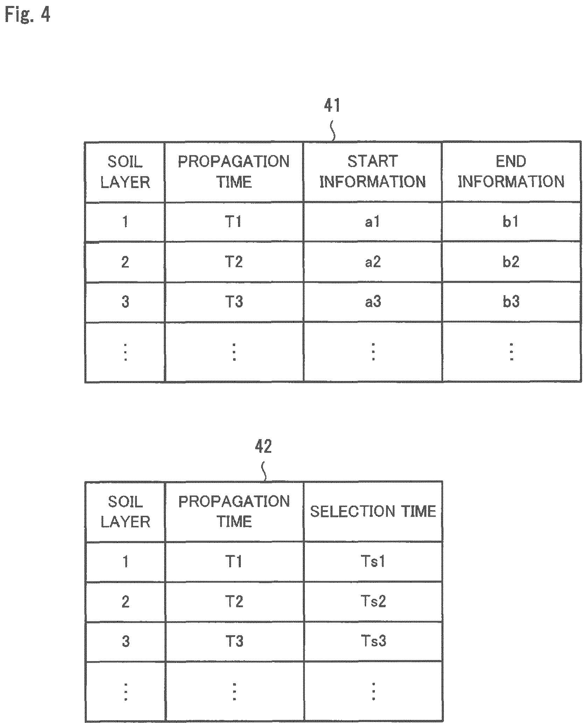

FIG. 4 is a diagram illustrating an example of a data structure of selection time period generation information. The above-described propagation time, start information, and end information regarding each soil layer are stored in the storage unit in advance such that the propagation time, start information, and end information are associated with each other as shown in selection time period generation information 41 in FIG. 4.

Alternatively, soil layers, propagation times, and selection time periods may be stored in the storage unit in advance in association with each other as shown in selection time period generation information 42 in FIG. 4.

Next, the selection unit 2 selects a piece of vibration information corresponding to the subject soil layer from among the acquired pieces of vibration information, using the selection time period. That is to say, the selection unit 2 selects the piece of vibration information measured in the selection time period corresponding to the subject soil layer.

The extraction unit 3 extracts a feature of vibrations, using the piece of vibration information thus acquired. Examples of features of vibrations include a resonance frequency.

Specifically, the extraction unit 3 first acquires reference vibration information (an input value x(t)), which serves as a reference corresponding to the vibrations applied by the vibrator 21 to soil, and a piece of vibration information (an output value y(t)) corresponding to the soil layer selected by the selection unit 2. Reference vibration information is obtained through experiments or simulations, and is stored in the storage unit.

Also, reference vibration information may be generated based on vibrations of the soil measured using a vibration meter other than the vibration meter 22 immediately after vibrations have been applied to soil.

Next, the extraction unit 3 calculates a function that expresses a relationship between the input value x(t) and the output value y(t) corresponding to the input value x(t). For example, the extraction unit 3 calculates a transfer function G(s). The transfer function G(s) is expressed as a ratio (Y(s)/X(s)) between an input value X(s) and an output value Y(s), which are obtained by applying Laplace transform to the input value x(t) and the output value y(t). Here, the ratio expresses how vibrations are transferred to the soil.

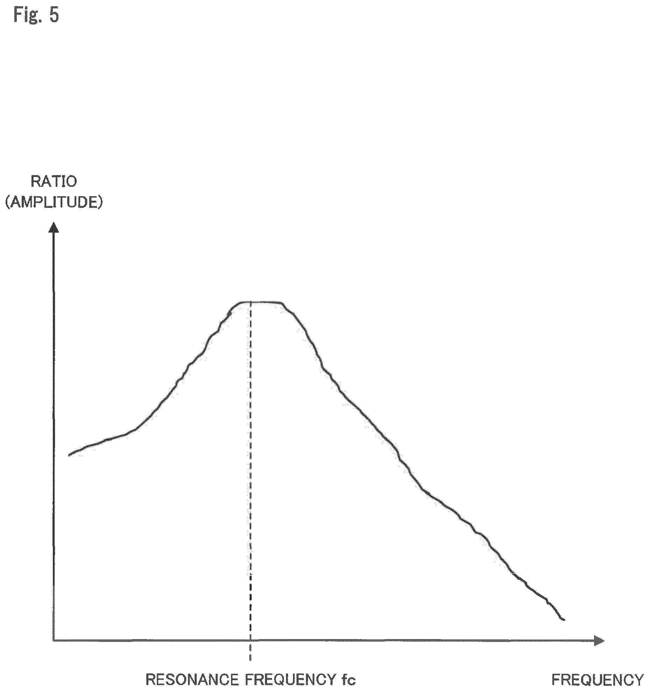

Next, using a relationship between the frequency corresponding to the vibrations (the frequency that has been swept) and the ratio (the amplitude), the extraction unit 3 extracts a frequency corresponding to the peak of the ratio as a feature of the vibrations. For example, the frequency corresponding to the peak of the ratio is regarded as a resonance frequency as shown in FIG. 5. FIG. 5 is a diagram illustrating the resonance frequency.

Note that the resonance frequency is used because the resonance frequency changes according to soil conditions and it is possible to estimate soil conditions by using such a relationship between the soil conditions and the resonance frequency.

Note that a feature of vibrations is not limited to that described above, and a feature other than the resonance frequency may be employed as a feature of vibrations. For example, information indicating the sharpness (the Q factor) of the resonance frequency curve may be employed as a feature of vibrations instead of the resonance frequency. Information indicating the sharpness of the resonance frequency curve is used because the sharpness of the resonance frequency curve changes according to soil conditions and it is possible to estimate soil conditions by using such a relationship between the soil conditions and the sharpness of the resonance frequency curve.

The estimation unit 4 estimates the soil conditions by referencing soil estimation information, using the extracted feature. Specifically, the estimation unit 4 first acquires the feature from the extraction unit 3. Next, using the acquired feature, the estimation unit 4 references soil estimation information, in which features and soil conditions are associated with each other, to estimate soil conditions. Soil estimation information has been stored in a storage unit (not shown) in advance, for example.

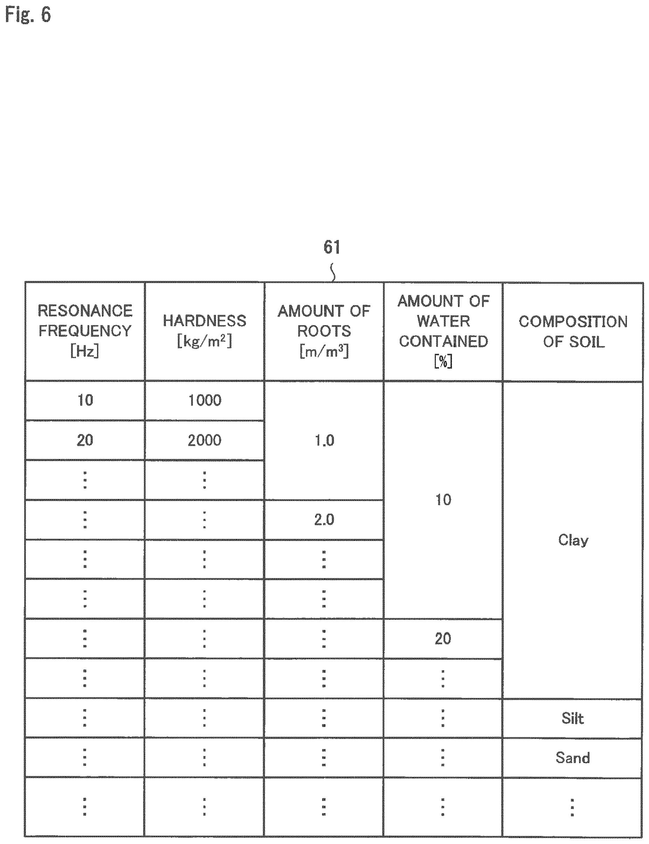

FIG. 6 is a diagram illustrating an example of a data structure of soil estimation information. The soil estimation information 61 shown in FIG. 6 is information in which the hardness (kg/m²) of soil, the amount (m/m³) of roots contained in soil, the amount (%) of water contained in soil, the composition of soil, or two or more of these elements are associated with a resonance frequency (Hz), which is a feature. Note that soil conditions are not limited to the above pieces of information.

The resonance frequency increases as the hardness of soil increases. This is because a hard object is resilient to an external force, and accordingly has a high resonance frequency. Also, the resonance frequency increases as the amount of roots contained in soil increases. This is because it is envisaged that the resonance frequency increases as the amount of roots increase if roots have a higher tension than soil, even though how the resonance frequency changes as the amount of roots increases depends on the type of roots and the type of soil. Another reason is that it is envisaged that the resonance frequency decreases as the amount of water contained in soil increases. This is because it is envisaged that fluids such as water generally have a lower tension than soil. Also, the resonance frequency varies according to the composition of soil (e.g. clay, mud, or sand).

Also, different factors may result in the same resonance frequency. For example, when the resonance frequency is 10 Hz, the combination of a hardness of 2000 (kg/m²), an amount of roots of 1.0 (m/m³), and an amount of water contained of 30 (%), and clay may be envisaged in addition to the combination of a hardness of 1000 (kg/m²), an amount of roots of 1.0 (m/m³), an amount of water contained of 10 (%), and clay. In order to obtain the hardness in such a case, factors other than the hardness may be determined by specifying the amount of roots, the amount of water, and the composition of soil, using a crop simulation, a moisture sensor, a soil analysis, and so on, and the hardness may be determined based on the results of specification.

Alternatively, the number of dimensions may be increased regarding the features of vibrations. When the number of dimensions is increased regarding the features of vibrations, the Q factor of the resonance frequency may also be specified as a feature amount, or a plurality of resonance frequencies may be measured, for example. As a result, even if different factors result in the same resonance frequency, it is possible to distinguish between soils that are in different conditions.

The output information generation unit 26 causes the output device 23 to output soil conditions corresponding to the soil layer. Specifically, the output information generation unit 26 first acquires information indicating soil conditions, from the estimation unit 4. Next, the output information generation unit 26 generates output information that is to be used to cause the output device 23 to output soil conditions corresponding to a soil layer, using information regarding the selected soil layer and the soil conditions corresponding to the soil layer. Thereafter, the output information generation unit 26 outputs the generated output information to the output device 23, and causes the output device 23 to output the soil conditions corresponding to the soil layer.

### [Apparatus Operations]

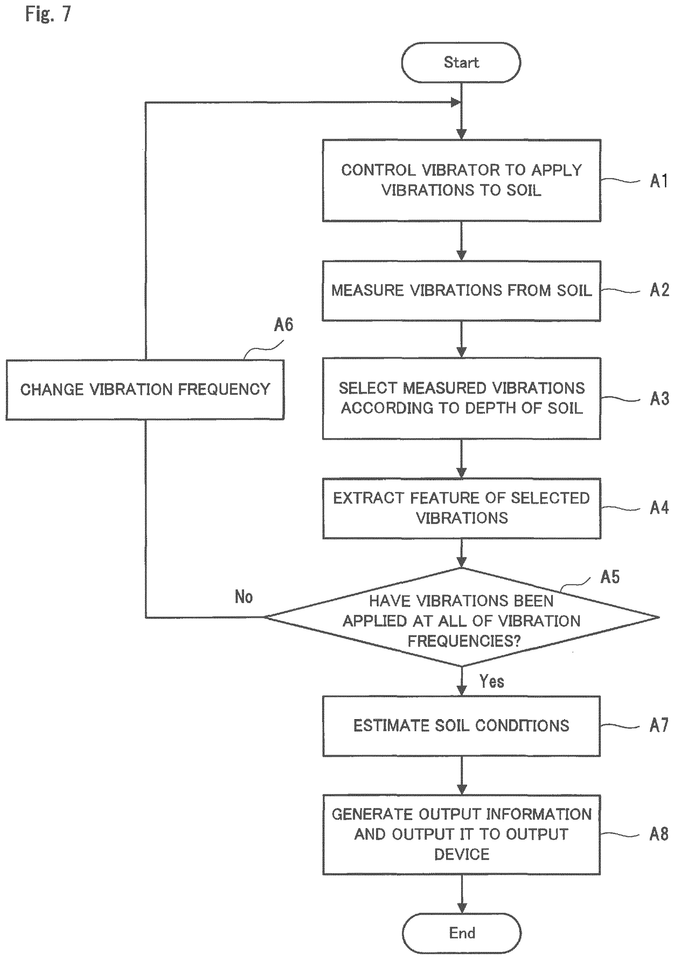

Next, operations of the soil condition estimation apparatus according to an exemplary embodiment of the present invention will be described with reference to FIG. 7. FIG. 7 is a diagram illustrating example operations of the soil condition estimation apparatus. In the following description, FIGS. 2 to 6 are referenced as appropriate. Also, in this exemplary embodiment, a soil condition estimation method is performed through operations of the soil condition estimation apparatus. Therefore, a description of a soil condition estimation method according to the present exemplary embodiment is substituted by the following description of operations of the soil condition estimation apparatus.

With reference to FIG. 7, the following describes a case in which soil conditions are estimated while the frequency to be applied is swept.

As shown in FIG. 7, first, in step A1, the control unit 24 controls the vibrator 21 to apply vibrations to soil. Specifically, in step A1, the control unit 24 transmits vibration setting information, which is used to set the vibration force and the vibration frequency, to the vibrator 21. Thereafter, the vibrator 21 causes the soil to vibrate, based on the received vibration setting information.

Subsequently, in step A2, the collecting unit 25 collects pieces of vibration information measured by the vibration meter 22 from soil. Specifically, in step A2, the collecting unit 25 first collects pieces of vibration information from the vibration meter 22 in time series. Next, the collecting unit 25 outputs the pieces of vibration information thus collected to the selection unit 2. Also, the collecting unit 25 stores the pieces of vibration information in a storage unit (not shown).

Next, in step A3, the selection unit 2 selects, by using the propagation time that vibrations take to propagate through soil, vibrations measured from soil, according to the depth of soil. That is to say, in step A3, the selection unit 2 selects a piece of vibration information corresponding to the subject soil layer from among the pieces of vibration information collected by the collecting unit 25, based on the propagation time of vibrations corresponding to the depth of the subject soil (soil layer).

Specifically, in step A3, the selection unit 2 first acquires the piece of vibration information from the collecting unit 25. Next, the selection unit 2 calculates a selection time period from the propagation time corresponding to the subject soil layer. For example, in a case in which the piece of vibration information corresponding to the soil layer 1 is selected, the calculation of a selection time period is performed using the propagation time T1 corresponding to the soil layer 1, to calculate the period of time (selection time period) from when vibrations are applied by the vibrator 21 to soil to when the vibrations 1 that have been reflected by the soil layer 1 can be measured by the vibration meter 22, using the propagation time T1 corresponding to the soil layer 1.

Next, the selection unit 2 selects the piece of vibration information corresponding to the subject soil layer from among the acquired pieces of vibration information, using the selection time period. That is to say, the selection unit 2 selects the piece of vibration information measured during the selection time period corresponding to the subject soil layer.

Note that, when the propagation time T1 is expressed as a period of time from the point in time t0, at which the ground surface of soil is caused to vibrate, to the point in time te1, at which the vibrations that have propagated through the soil layer 1 are measured at the ground surface, the selection time period ts1 corresponding to the soil layer 1 is a period of time from the point in time te1-a1, which is earlier than the point in time te1, to the point in time te1+b1, at which vibrations reflected by the soil layer 1 are no longer detectable.

The time al (start information) and the time b1 (end information) can be obtained through experiments or simulations, for example. Similarly, the selection time periods ts2, ts3, and so on are respectively calculated for the soil layers 2, 3, and so on other than the soil layer 1.

The above-described propagation time, start information, and end information regarding each soil layer are stored in the storage unit in advance such that the propagation time, start information, and end information are associated with each other as shown in the selection time period generation information 41 in FIG. 4.

Alternatively, soil layers, propagation times, and selection time periods may be stored in the storage unit in advance in association with each other as shown in the selection time period generation information 42 in FIG. 4.

Next, in step A4, the extraction unit 3 extracts a feature of the selected vibrations. Examples of features of vibrations include a resonance frequency.

Specifically, in step A4, the extraction unit 3 first acquires a piece of reference vibration information (the input value x(t)), which serves as a reference corresponding to the vibrations applied by the vibrator 21 to soil, and a piece of vibration information (the output value y(t)) corresponding to the soil layer selected by the selection unit 2.

Reference vibration information is obtained through experiments or simulations, and is stored in the storage unit. Alternatively, reference vibration information may be generated based on vibrations of soil measured using a vibration meter other than the vibration meter 22 immediately after vibrations have been applied to soil.

Next, in step A4, the extraction unit 3 calculates a function that expresses a relationship between the input value x(t) and the output value y(t) corresponding to the input value x(t). For example, the extraction unit 3 calculates a transfer function G(s).

The transfer function G(s) is expressed as a ratio (Y(s)/X(s)) between the input value X(s) and the output value Y(s), which are obtained by applying Laplace transform to the input value x(t) and the output value y(t). Here, the ratio expresses how vibrations are transferred to the soil.

Next, in step A4, using a relationship between the frequency corresponding to the vibrations (the frequency that has been swept) and the ratio (the amplitude), the extraction unit 3 extracts a frequency corresponding to the peak of the ratio as a feature of the vibrations (a resonance frequency). For example, the frequency corresponding to the peak of the ratio (the amplitude) is regarded as a resonance frequency as shown in FIG. 5. FIG. 5 is a diagram illustrating the resonance frequency.

Note that the resonance frequency is used because the resonance frequency changes according to soil conditions and it is possible to estimate soil conditions by using such a relationship between the soil conditions and the resonance frequency.

Note that a feature of vibrations is not limited to that described above, and a feature other than the resonance frequency may be employed as a feature of vibrations.

Next, in step A5, the control unit 24 determines whether or not the vibrator 21 has been caused to apply vibrations at all of the preset vibration frequencies. If the vibrator 21 has been caused to apply vibrations at all of the vibration frequencies (step A5: Yes), the control unit 24 performs processing to estimate soil conditions in step A7. If there is any vibration frequency at which vibrations have not been applied (step A5: No), the control unit 24 changes the vibration frequency in step A6. That is to say, the current vibration frequency is changed so as to be swept.

Next, in step A7, using the feature, the estimation unit 4 references soil estimation information, in which features and soil conditions are associated with each other, to estimate soil conditions. Specifically, in step A7, the estimation unit 4 first acquires the feature from the extraction unit 3. Next, using the acquired feature, the estimation unit 4 references soil estimation information, in which features and soil conditions are associated with each other, to estimate soil conditions. Soil estimation information has been stored in a storage unit (not shown) in advance, for example.

Soil estimation information is information in which the hardness (kg/m²) of soil, the amount (m/m³) of roots contained in soil, the amount (%) of water contained in soil, the composition of soil, or two or more of these elements are associated with a feature (e.g. a resonance frequency (Hz)). Note that soil conditions are not limited to the above pieces of information.

Next, in step A8, the output information generation unit 26 generates output information, using the estimated soil conditions, and outputs the output information to the output device 23.

Specifically, in step A8, the output information generation unit 26 first acquires information indicating soil conditions, from the estimation unit 4. Next, the output information generation unit 26 generates output information that is to be used to cause the output device 23 to output soil conditions corresponding to a soil layer, using information regarding the selected soil layer and the soil conditions corresponding to the soil layer. Thereafter, the output information generation unit 26 outputs the generated output information to the output device 23, and causes the output device 23 to output the soil conditions corresponding to the soil layer.

### [Effects of Present Exemplary Embodiment]

As described above, according to the present exemplary embodiment, it is possible to select vibrations according to the depth of soil, and therefore it is possible to estimate the conditions of soil at a desired depth (a soil layer), using the vibrations corresponding to the soil layer.

Also, according to the present exemplary embodiment, it is only required that measurement can be performed as shown in the gain diagram in FIG. 5. Therefore, it is possible to perform integration using a weak continuous wave. Using the vibrator 21, by intermittently applying weak vibrations to soil, or continuously applying vibrations to soil, it is possible to increase the S/N ratio of the vibrations that have been measured statistically. Therefore, it is possible to estimate soil conditions without damaging a ridge or the like of a soft soil such as a soil of a farm field.

Furthermore, it is unnecessary to apply a strong impact because the speed is not measured, and it is possible to provide the vibration meter 22 immediately next to the vibrator 21 to perform measurement. That is to say, with a conventional method through which the hardness is measured using the propagation speed of vibrations, it is necessary to detect a difference in speed, i.e. a difference in arrival time of vibrations. Also, it is necessary to separate the vibration source from the measurement point so that the time difference can be measured, and therefore it is necessary to apply strong vibrations. However, according to the present exemplary embodiment, it is unnecessary to detect the difference in speed, and it is only necessary that vibrations that have been intermittently or continuously applied to soil using the vibrator 21 are measured using the vibration meter 22, and the magnitude of the measured vibrations can be known. Therefore, it is possible to provide the vibration meter 22 immediately next to the vibrator 21 to perform measurement.

Also, it is possible to estimate soil conditions, and therefore it is possible to enable a worker to apply an appropriate treatment to the subject soil layer, by outputting soil conditions to the output device 23. Examples of an appropriate treatment include prompting a worker to plow soil if the soil is hard.

### [Program]

A program according to an exemplary embodiment of the present invention may be a program that causes a computer to execute steps A1 to A8 shown in FIG. 7. It is possible to realize the soil condition estimation apparatus and the coil condition estimation method according to the present exemplary embodiment by installing this program onto a computer and executing the program. If this is the case, the processor of the computer functions as the control unit 24, the collecting unit 25, the selection unit 2, the extraction unit 3, the estimation unit 4, and the output information generation unit 26, and performs processing.

Also, the program according to the present exemplary embodiment may be executed by a computer system that is constituted by a plurality of computers. If this is the case, for example, each computer may function as any of the control unit 24, the collecting unit 25, the selection unit 2, the extraction unit 3, the estimation unit 4, and the output information generation unit 26.

### [Physical Configuration]

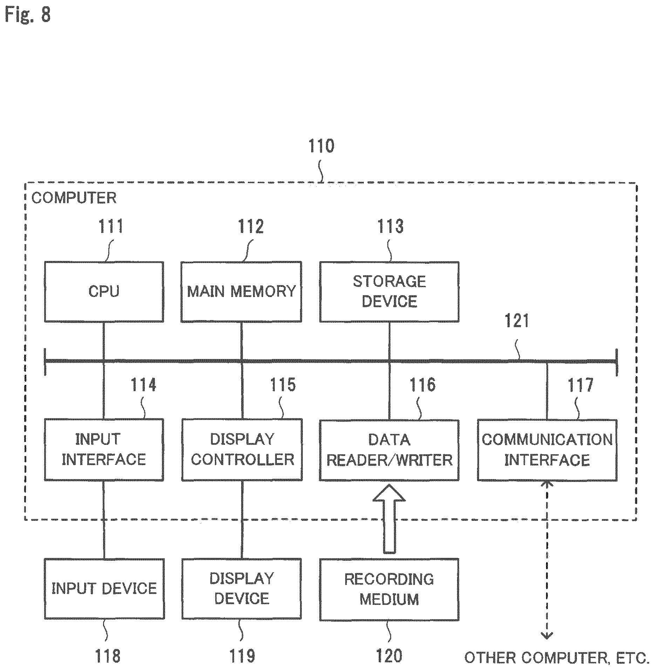

Here, a computer that executes a program according to an exemplary embodiment to realize a soil condition estimation apparatus will be described with reference to FIG. 8. FIG. 8 is a block diagram showing an example computer that realizes a soil condition estimation apparatus according to an exemplary embodiment of the present invention.

As shown in FIG. 8, a computer 110 includes a CPU 111, a main memory 112, a storage device 113, an input interface 114, a display controller 115, a data reader/writer 116, and a communication interface 117. These units are connected via bus 121 so as to be able to perform data communication with each other. Note that the computer 110 may include a GPU (Graphics Processing Unit) or a FPGA (Field-Programmable Gate Array) in addition to the CPU111 or instead of the CPU111.

The CPU111 loads a program (codes) according to the present exemplary embodiment stored in the storage device 113 to the main memory 112, and executes them in a predetermined order to perform various kinds of calculations. The main memory 112 is typically a volatile storage device such as a DRAM (Dynamic Random Access Memory). Also, the program according to the present exemplary embodiment is provided in the state of being stored in a computer-readable recording medium 120. Note that the program according to the present exemplary embodiment may be distributed on the Internet that is connected via the communication interface 117.

Specific examples of the storage device 113 include a hard disk drive, and a semiconductor storage device such as a flash memory. The input interface 114 mediates data transmission between the CPU 111 and the input device 118 such as a keyboard or a mouse. The display controller 115 is connected to a display device 119, and controls the display of the display device 119.

The data reader/writer 116 mediates data transmission between the CPU 111 and the recording medium 120, and reads out the program from the recording medium 120 and writes the results of processing performed in the computer 110 to the recording medium 120. The communication interface 117 mediates data transmission between the CPU 111 and another computer.

Specific examples of the recording medium 120 include general-purpose semiconductor storage devices such as a CF(Compact Flash (registered trademark)) and a SD (Secure Digital), a magnetic recording medium such as a flexible disk, and an optical recording medium such as a CD-ROM (Compact Disk Read Only Memory).

The following supplementary notes are also disclosed in relation to the above-described exemplary embodiment. Although at least one or all of the above-described exemplary embodiments can be expressed as, but are not limited to, Supplementary Note 1 to Supplementary Note 15 described below.

### (Supplementary Note 1)

A soil condition estimation apparatus comprising:
a selection unit that, by using a propagation time that vibrations take to propagate through a soil, selects vibrations measured from the soil, according the depth of the soil;
an extraction unit that extracts a feature of the selected vibrations; and
an estimation unit that estimates conditions of the soil by referencing soil estimation information in which the feature and conditions of the soil are associated with each other, using the feature.

### (Supplementary Note 2)

The soil condition estimation apparatus according to Supplementary Note 1,
wherein the selection unit selects vibrations corresponding to the depth of a subject soil, using a selection time period corresponding to the depth of the subject soil, the selection time period being used to select vibrations corresponding to the depth of the subject soil, and being calculated based on a preset propagation time.

### (Supplementary Note 3)

The soil condition estimation apparatus according to Supplementary Note 1 or 2,
wherein the propagation time is a period of time from when vibrations are applied to a ground surface of the subject soil to when the vibrations that have propagated through the soil are measured at the ground surface.

### (Supplementary Note 4)

The soil condition estimation apparatus according to any one of Supplementary Notes 1 to 3,
wherein the soil estimation information is information in which the hardness of a soil, the amount of roots contained in a soil, the amount of water contained in a soil, the composition of a soil, or two or more of these elements are associated with the feature.

### (Supplementary Note 5)

The soil condition estimation apparatus according to any one of Supplementary Notes 1 to 4,
wherein vibrations are applied to the soil, using a vibrator that is provided on a ground surface, and the vibrations are measured from the soil, using a vibration meter that is provided on the ground surface.

### (Supplementary Note 6)

A soil condition estimation method comprising:
(a) a step of, by using a propagation time that vibrations take to propagate through a soil, selecting vibrations measured from the soil, according the depth of the soil;
(b) a step of extracting a feature of the selected vibrations; and
(c) a step of estimating conditions of the soil by referencing soil estimation information in which the feature and conditions of the soil are associated with each other, using the feature.

### (Supplementary Note 7)

The soil condition estimation method according to Supplementary Note 6,
wherein in the (a) step, vibrations corresponding to the depth of a subject soil are selected using a selection time period corresponding to the depth of the subject soil, the selection time period being used to select vibrations corresponding to the depth of the subject soil, and being calculated based on a preset propagation time.

### (Supplementary Note 8)

The soil condition estimation method according to Supplementary Note 6 or 7,
wherein the propagation time is a period of time from when vibrations are applied to a ground surface of the subject soil to when the vibrations that have propagated through the soil are measured at the ground surface.

### (Supplementary Note 9)

The soil condition estimation method according to any one of Supplementary Notes 6 to 8,
wherein the soil estimation information is information in which the hardness of a soil, the amount of roots contained in a soil, the amount of water contained in a soil, the composition of a soil, or two or more of these elements are associated with the feature.

### (Supplementary Note 10)

The soil condition estimation method according to any one of Supplementary Notes 6 to 9,
wherein vibrations are applied to the soil, using a vibrator that is provided on a ground surface, and the vibrations are measured from the soil, using a vibration meter that is provided on the ground surface.

### (Supplementary Note 11)

A program recorded thereon, the program including instructions that causes a computer to carry out:
(a) a step of, by using a propagation time that vibrations take to propagate through a soil, selecting vibrations measured from the soil, according the depth of the soil;
(b) a step of extracting a feature of the selected vibrations; and
(c) a step of estimating conditions of the soil by referencing soil estimation information in which the feature and conditions of the soil are associated with each other, using the feature.

### (Supplementary Note 12)

The program according to Supplementary Note 11,
wherein in the (a) step, vibrations corresponding to the depth of a subject soil are selected using a selection time period corresponding to the depth of the subject soil, the selection time period being used to select vibrations corresponding to the depth of the subject soil, and being calculated based on a preset propagation time.

### (Supplementary Note 13)

The program according to Supplementary Note 11 or 12,
wherein the propagation time is a period of time from when vibrations are applied to a ground surface of the subject soil to when the vibrations that have propagated through the soil are measured at the ground surface.

### (Supplementary Note 14)

The program according to any one of Supplementary Notes 11 to 13,
wherein the soil estimation information is information in which the hardness of a soil, the amount of roots contained in a soil, the amount of water contained in a soil, the composition of a soil, or two or more of these elements are associated with the feature.

### (Supplementary Note 15)

The program according to any one of Supplementary Notes 11 to 14,
wherein vibrations are applied to the soil, using a vibrator that is provided on the ground surface, and the vibrations are measured from the soil, using a vibration meter that is provided on the ground surface.

As described above, according to the present invention, it is possible to estimate soil conditions according to the depth of soil. The present invention is useful in a technical field in which soil conditions need be estimated.

While the invention has been particularly shown and described with reference to exemplary embodiments thereof, the invention is not limited to these embodiments. It will be understood by those of ordinary skill in the art that various changes in form and details may be made therein without departing from the spirit and scope of the present invention as defined by the claims.

## Claims

1. A soil condition estimation apparatus comprising:
a selection means for, by using a propagation time that vibrations take to propagate through a soil, selecting vibrations measured from the soil, according the depth of the soil;
an extraction means for extracting a feature of the selected vibrations; and
an estimation means for estimating conditions of the soil by referencing soil estimation information in which the feature and conditions of the soil are associated with each other, using the feature.

2. The soil condition estimation apparatus according to claim 1,
wherein the selection means calculates a selection time period that is used to select vibrations corresponding to the depth of a subject soil, based on a preset propagation time, and selects vibrations corresponding to the depth of the subject soil, using the calculated selection time period.

3. The soil condition estimation apparatus according to claim 1 or 2,
wherein the propagation time is a period of time from when vibrations are applied to a ground surface of the subject soil to when the vibrations that have propagated through the soil are measured at the ground surface.

4. The soil condition estimation apparatus according to one of claims 1 to 3,
wherein the soil estimation information is information in which the hardness of a soil, the amount of roots contained in a soil, the amount of water contained in a soil, the composition of a soil, or two or more of these elements are associated with the feature.

5. The soil condition estimation apparatus according to one of claims 1 to 4,
wherein vibrations are applied to the soil, using a vibrator that is provided on the ground surface, and the vibrations are measured from the soil, using a vibration meter that is provided on the ground surface.

6. A soil condition estimation method comprising:
(a) a step of, by using a propagation time that vibrations take to propagate through a soil, selecting vibrations measured from the soil, according the depth of the soil;
(b) a step of extracting a feature of the selected vibrations; and
(c) a step of estimating conditions of the soil by referencing soil estimation information in which the feature and conditions of the soil are associated with each other, using the feature.

7. The soil condition estimation method according to claim 6,
wherein in the (a) step, a selection time period that is used to select vibrations corresponding to the depth of a subject soil is calculated, based on a preset propagation time, and vibrations corresponding to the depth of the subject soil are selected using the calculated selection time period.

8. The soil condition estimation method according to claim 6 or 7,
wherein the propagation time is a period of time from when vibrations are applied to a ground surface of the subject soil to when the vibrations that have propagated through the soil are measured at the ground surface.

9. The soil condition estimation method according to any one of claims 6 to 8,
wherein the soil estimation information is information in which the hardness of a soil, the amount of roots contained in a soil, the amount of water contained in a soil, the composition of a soil, or two or more of these elements are associated with the feature.

10. The soil condition estimation method according to one of claims 6 to 9,
wherein vibrations are applied to the soil, using a vibrator that is provided on the ground surface, and the vibrations are measured from the soil, using a vibration meter that is provided on the ground surface.

11. A program including instructions that causes a computer to carry out:
(a) a step of, by using a propagation time that vibrations take to propagate through a soil, selecting vibrations measured from the soil, according the depth of the soil;
(b) a step of extracting a feature of the selected vibrations; and
(c) a step of estimating conditions of the soil by referencing soil estimation information in which the feature and conditions of the soil are associated with each other, using the feature.

12. The program according to claim 11,
wherein in the (a) step, a selection time period that is used to select vibrations corresponding to the depth of a subject soil is calculated, based on a preset propagation time, and vibrations corresponding to the depth of the subject soil are selected using the calculated selection time period.

13. The program according to claim 11 or 12,
wherein the propagation time is a period of time from when vibrations are applied to a ground surface of the subject soil to when the vibrations that have propagated through the soil are measured at the ground surface.

14. The program according to one of claims 11 to 13,
wherein the soil estimation information is information in which the hardness of a soil, the amount of roots contained in a soil, the amount of water contained in a soil, the composition of a soil, or two or more of these elements are associated with the feature.

15. The program according to one of claims 11 to 14,
wherein vibrations are applied to the soil, using a vibrator that is provided on the ground surface, and the vibrations are measured from the soil, using a vibration meter that is provided on the ground surface.
